# EUROPEAN PATENT SPECIFICATION

(11) **EP 2 517 682 B1**
(45) Date of publication and mention of the grant of the patent: **18.10.2017**
(21) Application number: 10839452.9
(22) Date of filing: 22.12.2010
(51) Int. Cl.: A61F 13/15, A61F 13/511, A61F 13/53, A61F 13/539

(54) **ABSORPTIVE ARTICLE**
SAUGFÄHIGER ARTIKEL
ARTICLE ABSORBANT

(30) Priority: 25.12.2009 JP 2009294477
(43) Date of publication of application: 31.10.2012
(73) Proprietor: Daio Paper Corporation, Ehime 799-0492 (JP)
(72) Inventor: KURAMOCHI, Mihoko, Sakura-shi Tochigi 329-1411 (JP)
(74) Representative: Schön, Christoph
(86) International application number: PCT/JP2010/073128
(87) International publication number: WO 2011/078222

(56) References cited:
- WO-A1-2007/061035
- WO-A1-2009/041223
- WO-A1-2009/139248
- JP-A- H10 201 788
- JP-A- 2002 291 806
- JP-A- 2003 033 397
- JP-A- 2007 195 665
- JP-A- 2009 082 480
- JP-A- 2009 273 724

## Description

### Technical Field

The present invention relates to an absorptive article such as a sanitary napkin, a pantiliner, an incontinence pad and the like for absorbing menstrual blood, vaginal discharge and the like, or more particularly to an absorptive article which fits the shape and movement of a body, eliminates wrinkles and improves a wearing feeling.

### Background Art

Hitherto, as absorptive articles such as a sanitary napkin, a pantiliner, an incontinence pad and the like, those interposing an absorptive element containing an absorbing body made of a cotton-state pulp between an impermeable back-surface sheet such as a polyethylene sheet, a polyethylene laminate unwoven cloth or the like and a permeable front-surface sheet such as a nonwoven cloth, a permeable plastic sheet or the like.

Various improvements have been made for this type of absorptive article, and in order to improve a wearing feeling and fitting performances, those provided with an emboss having a predetermined shape from the front surface side of the permeable front-surface sheet are known.

For example, Patent Document 1 below discloses, as illustrated in Fig. 5, a sanitary napkin 50 in which a front-surface sheet 51, an absorbing core 52, and a back-surface sheet 53 are laminated in this order. A first emboss 54 drawing a line going in the front-and-rear direction is given on both side end portions and on a center portion in the front-and-rear direction of the absorbing core 52 from above the front-surface sheet 51, and a second emboss 55 drawing a straight line starting from the center portion in the width direction as a base end to the both sides outward in the front-and-rear direction, respectively, from this base end is given on at least one of a front end portion and a rear end portion of the absorbing core 52 from above the front-surface sheet 51. According to the sanitary napkin 50 as above, if a pressure P going inward from the both sides in the width direction is applied in a wearing state, upper layer portions of the front-surface sheet 51 and the absorbing core 52 are brought closer to a center line C side in the width direction (direction indicated by reference character F) from the second emboss 55 side. As a result, particularly at least the surface shape of a center portion T in the width direction on the end portion in the front-and-rear direction of the sanitary napkin 50 protrudes to the wearer side, and the fitting performance for the wearer is considered to be improved.

Patent Document 2 below discloses an absorptive article 60, as illustrated in Fig. 6, in which left and right grooves 61 and 62 are separated from both a front groove 63 and a rear groove 64, and front end portions 61A and 62A of the left and right grooves are located closer to the front side in the longitudinal direction than a rear end portion 63A of the front groove, while rear end portions 61B and 628 of the left and right grooves are located closer to the back side in the longitudinal direction than a front end portion 64A of the rear groove. According to such absorptive article 60, if an area surrounded by the left and right grooves 61 and 62 is subjected to a compression force inward in the width direction from right and left in the worn state, the article is stably raised toward the skin side of the wearer using a substantially V-shaped folding line 65 formed of lines virtually extended from the left and right grooves 61 and 62 to the front end portion side and a substantially V-shaped folding line 66 formed of lines virtually extended from the left and right grooves 61 and 62 to the rear end portion side as flexible axes and fits the excretion portion of the wearer.

### Citation List

### Patent Literature

Patent Document 1: Japanese Unexamined Patent
Application Publication No. 2009-82480
Patent Document 2: Japanese Unexamined Patent
Application Publication No. 2007-195665

JP 2003-033397 A discloses a sanitary napkin comprising an absorption body supported with a liquid impermeable type backsheet, wherein a region other than a medium convex section has on almost entire face thereof a number of perfect circle-shaped embossed concave sections for pressing.

WO 2009/139248 A1 discloses an absorbent article wherein the contact area of an absorber with the skin is wide and the central part of an absorption layer can always obtain a stabilized deformation state while preventing occurrence of kink.

JP H10-201788 A discloses an absorbent article comprising a next-to-skin permeable surface, a nonpermeable surface in a state of non-contact with the skin, and an absorbent part interposed between the two surfaces, said absorbent article being formed essentially in an oblong shape.

WO 2009/041223 A1 discloses a sanitary napkin which comprises a front face sheet, a back face sheet and a liquid-absorbing structure located between the front sheet and the back sheet, wherein a backward compressed groove is formed in the back part along the lengthwise direction of the vertically long sanitary napkin in the body-contact side of the sanitary napkin.

JP 2002-291806 A discloses an absorbable article comprising an absorber interposed between a liquid permeable front surface sheet and a liquid impermeable rear surface sheet. In this article, linear embosses gradually approaching to a centerline in the longitudinal direction of the article toward end edge sides of the article are respectively formed at both sides by striding over the centerline in the longitudinal direction of the article on a front end part region and a rear end part region at the front surface side of the article.

WO 2007/061035 A1 discloses an absorbent article which comprises a liquid-permeable surface sheet, a back face sheet and an absorbent located between them, and has a pair of right and left lengthwise embossed channels formed almost in the longitudinal direction in the liquid-permeable surface sheet side, wherein the lengthwise embossed channels are in a tapered shape with the distance between them narrowing from the back side toward the front side of the absorbent article.

### Summary of Invention

### Technical Problem

The shape of a female body is very different depending on the portion such as a recess is formed in a blood discharge opening portion and a perineal region, respectively, in the center part of the body, the front side from this center part is rounded around pubes, and a valley in the buttocks is formed in the back side from the center part. When movement of the body is examined while walking, the center part of the body rarely moves since it is a portion close to the axis of the body, the back side from this center part has the largest movement since the buttocks vertically move during walking, and the front side from the center part has relatively less movement than the back side but some movement is generated therein. As described above, the shape of the body and the movement of the body are very different depending on each portion, and thus, it is important that the absorptive article is made deformable so as to adapt to the shape and movement of the body, while the movement of each portion is not allowed to interlock with the other portions or particularly the center part corresponding to the blood discharge opening portion in order to improve fitting performance and wearing feeling of the absorptive article.

However, with the absorptive articles 50 and 60 in the above-described Patent Documents 1 and 2, despite the fact that the shape and movement of the body are different between the front side and the back side as described above, the embosses are given substantially symmetrically in the longitudinal direction. If the front side portion and the back side portion are formed so as to be easily deformed to follow the valley in the buttocks, the fitting performance on the front side portion is lost, the movement of the front side portion cannot be followed, and front leakage along the curve around the pubes can easily occur, while if the front side portion and the back side portion are formed so as to be easily deformed to follow the curve around the pubes to the contrary, the fitting performance on the back side portion is lost, the movement of the buttocks cannot be followed, and rear leakage down the valley of the buttocks can easily occur. Moreover, wrinkles can be generated even in the center part corresponding to the blood discharge opening portion with the movement of the buttocks of the back side portion with particularly strenuous movement or a close contact state with the blood discharge opening portion or perineal region can be released, which might deteriorate the wearing feeling.

Moreover, if the portion surrounded by the pair of left and right embosses 54 and 54 (61 and 62) is subjected to a compression force inward in the width direction from the right and left, since the emboss is given substantially symmetrically in the longitudinal direction in the center part corresponding to the blood discharge opening portion, the center of this portion is raised. However, the shape of the blood discharge opening portion of a woman is actually complicated, and even if the portion can be made to substantially fit the periphery of the blood discharge opening portion of a woman, since the perineal region from the vaginal opening to the periphery of the anus on the back side forms a three-dimensional shape in the small recessed state, the portion cannot fit this perineal region. Therefore, menstrual blood or the like that cannot be absorbed in the periphery of the blood discharge opening is not absorbed in the perineal region, either, and might cause rear leakage.

A main object of the present invention is to provide an absorptive article in which the fitting performance is improved by following the shape and movement of the body and wrinkles in a blood discharge opening corresponding portion are eliminated to improve the wearing feeling by separating movement of each part from each other so that the blood discharge opening corresponding portion is not affected.

### Solution to Problem

As the present invention according to claim 1 in order to solve the above problems, an absorptive article is provided in which an absorbing element is interposed between a permeable front-surface sheet and an impermeable back-surface sheet, characterized in that a pair of right and left embossed portions corresponding to a blood discharge opening is formed by an arc-shaped curve along a substantially longitudinal direction of the absorptive article and having a center of curvature on the center line side in the longitudinal direction on each of both sides of the longitudinal center line, on a portion corresponding to the blood discharge opening which portion corresponds to a blood discharge opening portion and a perineal region of a wearer when wearing, a front side embossed portion in a substantially width direction of the absorptive article is formed on a front side portion located on the front side with a separation width provided in the longitudinal direction of the absorptive article from the embossed portion corresponding to the blood discharge opening, a discontinuous zone of the embossed line where an embossed line is separated in the longitudinal direction between the embossed portion corresponding to the blood discharge opening and the front side embossed portion by the separation width, is formed across a width direction of the absorptive article, and a back side embossed portion having a substantially inverted V-shape gradually expanding rearward to both sides from a position on the longitudinal center line, is formed on a back side portion located on the back side with a separation width provided in the longitudinal direction of the absorptive article from the embossed portion corresponding to the blood discharge opening, a discontinuous zone of the embossed line where an embossed line is separated in the longitudinal direction between the embossed portion corresponding to the blood discharge opening and the back side embossed portion by the separation width, is formed across a width direction of the absorptive article; and the pair of right and left embossed portions corresponding to the blood discharge opening has front end portions and rear end portions provided separately from each other on both sides of the longitudinal center line, respectively, and the separation width between the rear end portions is smaller than the separation width between the front end portions, and the front side embossed portion is formed by an arc-shaped curve having a center of curvature on the side closer to the front side of the absorptive article than the front side embossed portion, an angle formed by tangent lines of both right and left end portions is 90° or more and embossed lines of the back side embossed portion which extend on both sides from the position on the longitudinal center line to the back side, have a curved shape having a center of curvature at an outer side of the absorptive article.

In the above-described invention described in claim 1, the embosses for facilitating deformation to adapt to the shape and movement of each part of the body are formed on the blood discharge opening corresponding portion corresponding to the blood discharge opening portion and the perineal region of the wearer in wearing, on the front side portion located on the front side with the separation width provided in the longitudinal direction of the absorptive article from this blood discharge opening corresponding portion, and on the back side portion located on the back side with the separation width provided in the longitudinal direction of the absorptive article from the blood discharge opening corresponding portion.

Specifically, on the blood discharge opening corresponding portion, the blood discharge opening corresponding portion embosses having the arc-shaped curve in the substantially longitudinal direction of the absorptive article and having the center of curvature on the longitudinal center line are formed, respectively, on the both sides of the longitudinal center line. Particularly, in the present invention, the front end portions and the rear end portions in the pair of right and left blood discharge opening corresponding portion embosses are provided separately from each other on the both sides of the longitudinal center line, respectively, and the separation width between the rear end portions is set smaller than the separation width between the front end portions. Therefore, the front side in the portion surrounded by the blood discharge opening corresponding portion embosses is raised to the front surface side in a relatively wide range in the width direction by a pressure inward from the both sides in the width direction from the femora, and thus, the front side suitably fits the blood discharge opening portion. Moreover, since the back side is raised to the front surface side within a relatively narrow range in the width direction, the back side suitably fits the perineal region. Moreover, since the front side embossed portion in the substantially width direction of the absorptive article is formed on the front side portion, the front side portion can easily deform along the curve around the pubes with this front side embossed portion as the flexible axis. Furthermore, since the back side embossed portion having the substantially inverted V-shape gradually expanding rearward to the both sides from the position on the longitudinal center line is formed on the back side portion, the back side portion can easily deform along the valley of the buttocks with this back side embossed portion as the flexible axis. Therefore, since the article is deformed in compliance with the shape of each part of the body, fitting performance is improved, and leakage of menstrual blood or the like can be reliably prevented.

Each of the blood discharge opening corresponding portion, the front side portion, and the back side portion is deformed so as to fit the body as described above and a discontinuous zone of the embossed line is formed between the blood discharge opening corresponding portion and the front side portion as well as the back side portion, respectively. Therefore, this discontinuous zone functions as a separating zone which separates movement of the front side portion from that of the back side portion and relaxes an influence on the blood discharge opening corresponding portion, the fitting performance of the blood discharge opening corresponding portion to the body is stably maintained regardless of the movements of the front side portion and the back side portion, wrinkles in the blood discharge opening corresponding portion are eliminated, and the wearing feeling is improved.

When the right and left legs are moved back and forth alternately as in walking, the front side portion and the back side portion are interlocked with the movements of the right and left legs and generate a movement like twisting deformation. However, since the center parts in the width direction of the discontinuous zones become the base ends of the twisting deformation of the front side portion and the back side portion and function as separating base points which separate the movement of the front side portion from that of the back side portion, respectively, and relax the influences to the blood discharge opening corresponding portion, deformation of the blood discharge opening corresponding portion caused by interlocking with the twisting deformation of the front side portion and the back side portion and wrinkles are suppressed, and the wearing feeling can be improved.
Furthermore, in the absorptive article described in claim 1 the front side embossed portion is formed by an arc-shaped curve having the center of curvature on the side closer to the front side of the absorptive article than the front side embossed portion and an angle formed by tangent lines of both right and left end portions is 90° or more.
In the invention described in claim 1, in order to make the front side portion easily deformable with the shape following the curve around the pubes of the wearer by using the front side embossed portion as a flexible axis, the front side embossed portion is formed of a curve having the center of curvature on the side closer to the front of the absorptive article than the front side embossed portion, and the angle formed by tangent lines of both right and left end portions is 90° or more.

As the present invention according to claim 2, the absorptive article described in claim 1 is provided wherein in the substantially inverted V-shape embossed portion (12) constituting the back side embossed portion each of the embossed lines (12a and 12b) formed on both sides on the longitudinal center line (CL) is formed such that a virtual line (K1 or K2) extended from the front end portion of the embossed line on one side to the other side across the longitudinal center line (CL) becomes substantially parallel with a tangent line (S1 or S2) of the rear end portion of the embossed line (10a) formed on the other side of the longitudinal center line (CL) of the embossed portion corresponding to the blood discharge opening (10).

In the above-described invention described in claim 2, regarding the back side portion where the movement of the body is relatively larger than that of the front side portion, in addition to the formation that the rear-end separation width is smaller than the front-end separation width of the pair of right and left embossed portions corresponding to a blood discharge opening as in the invention described in claim 1, in the substantially inverted V-shape embossed portion constituting the back side embossed portion each of the embossed lines formed on the both sides on the longitudinal center line is formed such that the virtual line extended from the front end portion of the embossed line on one side (V-shaped top portion at the center) to the other side across the longitudinal center line and the tangent line of the rear end portion of the embossed line formed on the other side of the longitudinal center line of the embossed portion corresponding to the blood discharge opening become substantially parallel with each other. As a result, the back side portion is deformed along the valley of the buttocks, and the wrinkles having developed in the direction of the blood discharge opening corresponding portion from the front end portion can be absorbed by the embossed portions corresponding to the blood discharge opening , wrinkles are no longer formed in the portions surrounded by the embossed portions and the blood discharge opening corresponding portion can be made to fit the blood discharge opening corresponding portion and the perineal region more reliably.

As the present invention according to claim 3, the absorptive article described in any one of claims 1 to 2 is provided in which, in the back side embossed portion, the angle formed by the emboss lines formed on the both sides across the longitudinal center line is less than 90°.

In the invention described in claim 3, in order to make the back side portion easily deformable with the shape following the valley in the buttocks of the wearer by using the back side embossed portion as a flexible axis, the back side embossed portion is configured such that the angle formed by the embossed lines formed on the both sides across the longitudinal center line is less than 90°.

### Advantageous Effects of Invention

As described above in detail, according to the present invention, the fitting performance is improved by following the shape and the movement of the body, and wrinkles in the blood discharge opening corresponding portion are eliminated and the wearing feeling can be improved by separating movement of each part from each other so that the blood discharge opening corresponding portion is not affected.

### Brief Description of Drawings

Fig. 1 is a partially broken extended diagram of a sanitary napkin 1 according to the present invention.
Fig. 2 is an explanatory diagram of embosses.
Fig. 3 is an enlarged diagram of the essential part thereof.
Fig. 4 is an extended diagram of the sanitary napkin 1 according to another embodiment.

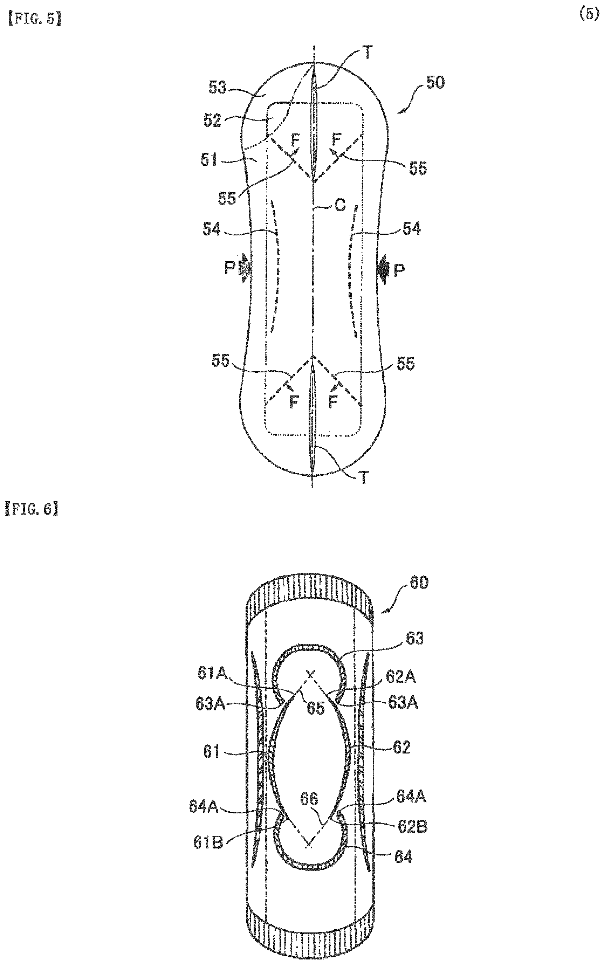

Fig. 5 is an extended diagram of a prior-art sanitary napkin 50.
Fig. 6 is an extended diagram of a prior-art sanitary napkin 60.

### Description of Embodiments

An embodiment of the present invention will be described below in detail by referring to the attached drawings.

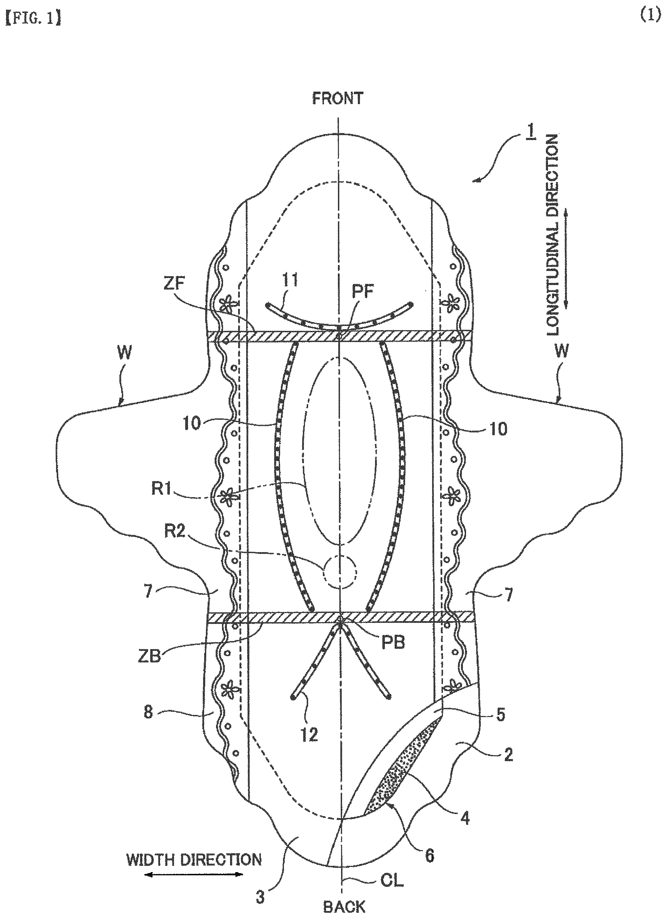

A sanitary napkin 1 according to the present invention includes, as illustrated in Fig. 1, an impermeable back-surface sheet 2 made of a polyethylene sheet, a polypropylene sheet or the like, a permeable front-surface sheet 3 which has menstrual blood, vaginal discharge and the like quickly permeate, an absorbing element 6 including an absorbing body 4 made of a cotton-state pulp or synthetic pulp interposed between these both sheets 2 and 3 and crepe paper 5 surrounding the absorbing body 4 for maintenance of the shape and improvement of diffusion performance of this absorbing body 4, and side unwoven cloths 7 and 7 formed in the longitudinal direction each on the both side portions of the front surface. In the periphery of the absorbing body 4, outer edge portions of the impermeable back-surface sheet 2 and the permeable front-surface sheet 3 are bonded to the upper and lower end edge portions thereof by an adhesive such as hot-melt or bonding means such as heat-seal or the like, the impermeable back-surface sheet 2 and the side unwoven cloths 7 protruding to the sides than the absorbing body 4 are bonded on the both side edge portions thereof by the adhesive such as hot-melt or the bonding means such as heat-seal or the like, and a flap portion 8 where the absorbing body 4 is not present is formed on an outer periphery.

A structure of the sanitary napkin 1 will be further described below in detail.

A sheet material having at least water shielding performance such as an olefin resin sheet including polyethylene, polypropylene and the like is used for the impermeable back-surface sheet 2. Other than the above, a laminate unwoven cloth in which an unwoven cloth is laminated on the polyethylene sheet or the like, an unwoven cloth sheet in which impermeability is substantially ensured by providing a water-proof film therein (in this case, the impermeable back-surface sheet is formed of the water-proof film and the unwoven cloth) and the like can be also used. In recent years, those with moisture permeability tend to be used from the viewpoint of prevention of sultry feeling. This water-shielding/moisture permeable sheet material is a micro-porous sheet obtained by melting and kneading inorganic filler in an olefin resin such as polyethylene, polypropylene and the like and molding a sheet and then, by drawing it in an uniaxial or biaxial direction.

Subsequently, a porous or non-porous unwoven cloth or a porous plastic sheet is suitably used for the permeable front-surface sheet 3. As material fibers constituting the unwoven cloth, in addition to synthetic fibers including olefins such as polyethylene, polypropylene and the like and polyesters, polyamide and the like, recycled fibers such as rayon, cupra and the like, and natural fibers such as cotton and the like can be used, and unwoven cloth obtained by appropriate processing methods such as spunrace method, spun-bond method, thermal bond method, melt-blown method, needle punch method and the like can be used. Among these processing methods, the spunrace method is excellent in its rich flexibility and drape property, while the thermal bond method is excellent in bulkiness and softness.

The absorbing body 4 interposed between the impermeable back-surface sheet 2 and the permeable front-surface sheet 3 is formed of fluff pulp and water-absorbing polymer, for example. The water-absorbing polymer is mixed in the pulp constituting the absorbing body as granular powder, for example. The pulp includes chemical pulp obtained from lumber, cellulose fiber such as molten pulp or the like, artificial cellulose fiber such as rayon, acetate or the like, and softwood pulp with a fiber length longer than that of hardwood pulp is preferably used in terms of functions and price.

For the absorbing body 4, synthetic fibers may be mixed. As the synthetic fibers, olefins such as polyethylene, polypropylene and the like, polyesters such as polyethylene terephthalate, polybutylene terephthalate and the like, polyamides such as nylon and copolymers thereof can be used or two kinds of them may be mixed. Moreover, complex fibers including core-clad fibers having a fiber with a high melting point as the core and a fiber with a low melting point as the clad, side-by-side fibers, split-type fibers can be also used. In the case of the above-described synthetic fibers, those subjected to surface treatment by a hydrophilic agent are preferably used for hydrophobic fibers so as to give affinity for body fluid.

If the crepe paper 5 surrounding the absorbing body 4 is provided as in this example, the crepe paper 5 is interposed between the permeable front-surface sheet 3 and the absorbing body 4 in the end, and the body fluid is quickly diffused by the crepe paper 5 with excellent absorbency and backf low of the menstrual blood and the like can be prevented.

On the other hand, the side unwoven cloths 7 and 7 are provided on both side portions on the front surface of this sanitary napkin 1 in the longitudinal direction over the substantially whole length of the napkin 1. Parts of the side unwoven cloths 7 and 7 are extended to the sides, and these form wing-shaped flaps W and W with a part of the impermeable back-surface sheet 2 similarly extended to the sides.

For the side unwoven cloth 7, a water-repellent treated unwoven cloth or hydrophilically treated unwoven cloth can be used from the viewpoint of a function to be emphasized. If an emphasis is to be placed on a function to prevent permeation of menstrual blood, vaginal discharge and the like or to improve a feeling on the skin, for example, the water-repellent treated unwoven cloth coated with silicon, paraffin, alkyl chromic chloride water-repellent or the like is preferably used. If an emphasis is placed on absorbency of menstrual blood or the like in the wing-shaped flaps W and W, the synthetic fibers are made swollen or porous by using a method of polymerization through coexistence of a compound having a hydrophilic group such as an oxidized product of polyethylene glycol, for example in a manufacture process of the synthetic fiber, a method of precipitating a hydroxide of metal through treatment with metal salt such as stannic chloride, partial melting of the surface so as to make it porous and the like so that a hydrophilically treated unwoven cloth having a hydrophilic property by applying a capillary phenomenon is preferably used.

### (Emboss structure)

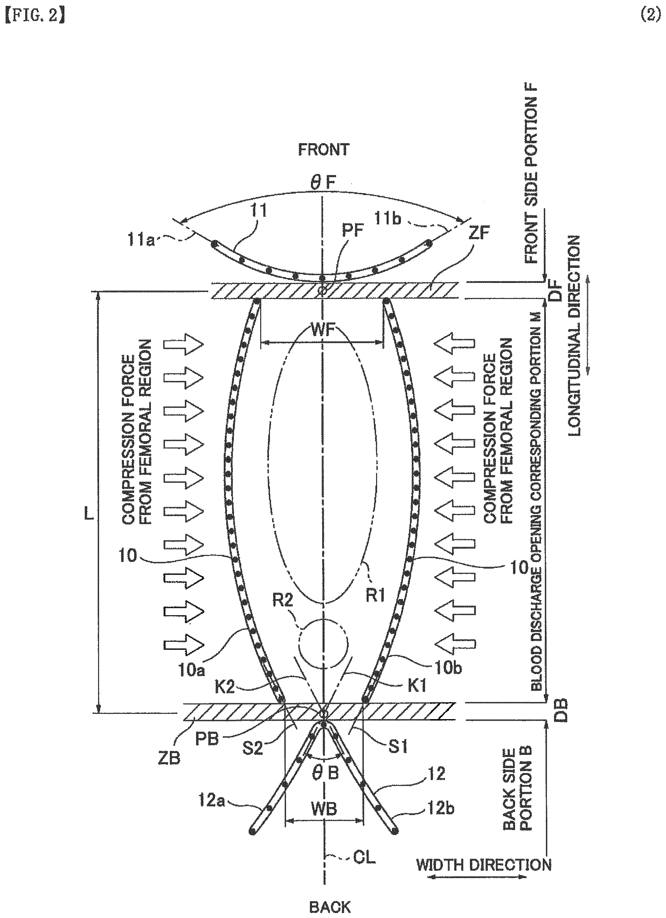

In the sanitary napkin 1, as illustrated in Fig. 2, embosses 10 to 12 having different shapes adapting to the shape and movement of each part of the body so as to make the sanitary napkin deformable are formed in a blood discharge opening corresponding portion M corresponding to a blood discharge opening portion and a perineal region of a wearer when wearing, a front side portion F located on the front side with a separation width DF provided in the napkin longitudinal direction from this blood discharge opening corresponding portion M, and a back side portion B located on the back side with a separation width DB provided in the napkin longitudinal direction from the blood discharge opening corresponding portion M, respectively.

An embossed portion corresponding to a blood discharge opening 10 having an arc-shaped curve in the substantially longitudinal direction of the sanitary napkin 1 and having a center of curvature on a center line side in the longitudinal direction of the napkin is formed on the blood discharge opening corresponding portion M, each on both sides of the longitudinal center line CL, a front side embossed portion 11 in the substantially width direction of the sanitary napkin 1 is formed on the front side portion F with separation from the embossed portions corresponding to the blood discharge opening 10, and a back side embossed portion 12 having a substantially inverted V-shape gradually expanding rearward to the both sides from a position (PB) on the longitudinal center line CL is formed on the back side portion B with separation from the embossed portions corresponding to the blood discharge opening 10. Moreover, the pair of right and left embossed portions corresponding to the blood discharge opening 10 has front end portions and rear end portions provided separately from each other on the both sides of the longitudinal center line, respectively, and a separation width WB between the rear end portions is smaller than the separation width WF between the front end portions.

As a result, the front side in the portion surrounded by the embossed portions corresponding to the blood discharge opening 10 and 10 is raised to the front surface side in a relatively wide range in the width direction by a inward pressure from the both sides in the width direction from the femora in the blood discharge opening corresponding portion M. Therefore, the front side suitably fits a blood discharge opening portion R1, while since the back side is raised to the front surface side within a relatively narrow range in the width direction, the back side suitably fits a perineal region R2. Moreover, the front side portion F can easily deform along the curve around the pubes with the front side embossed portion 11 as the flexible axis and suitably fits the front side of the body. Furthermore, the back side portion B can easily deform along the valley of the buttocks with the back side embossed portion 12 as the flexible axis and suitably fits the back side of the body. As described above, since each of the blood discharge opening corresponding portion M, the front side portion F, and the back side portion B is deformed along the shape of the body and fits the surface of the body without a gap, a wearing feeling and fitting performance are improved, as well as leakage of menstrual blood or the like can be reliably prevented.

In addition, since each portion of the sanitary napkin 1 is deformed along the shape of the body so as to be brought into close contact with the surface of the body and discontinuous zones ZF and ZB of the embossed line where the embossed line is separated in the longitudinal direction are formed in the width direction between the blood discharge opening corresponding portion M and the front side portion F as well as the back side portion B, respectively, each of the discontinuous zones ZF and ZB functions as a separating zone which prevents movement of the front side portion F and the back side portion B caused by movement of the body from being interlocked with the blood discharge opening corresponding portion M but separates them from each other, and the fitting performance of the blood discharge opening corresponding portion M to the body is stably maintained regardless of the movements of the front side portion F and the back side portion B.

Moreover, in this sanitary napkin 1, when the right and left legs are moved back and forth alternately as in walking, the front side portion F and the back side portion B are interlocked with the movements of the right and left legs and generate a twisting deformation. However, since the center parts in the width direction of the discontinuous zones ZF and ZB function as separation base points PF and PB, respectively, the front side portion F and the back side portion B are twisted and deformed so as to oscillate around the longitudinal center line CL with the separation base points PF and PB as base ends, respectively. Thus, the movements of the front side portion F and the back side portion B are not interlocked with the blood discharge opening corresponding portion M, and deformation and wrinkles in the blood discharge opening corresponding portion M can be suppressed.

As illustrated in Fig. 2, in this sanitary napkin 1, in embossed lines 12a and 12b formed on the both sides on the longitudinal center line CL and constituting the back side embossed portion 12, the embossed line 12a formed on the left side of the longitudinal center line CL is formed such that a virtual line K1 extended to the other side across the longitudinal center line CL from the front end portion becomes substantially parallel with a tangent line S1 of the rear end portion of an embossed line 10b formed on the right side of the longitudinal center line CL in the embossed portions corresponding to the blood discharge opening 10. Similarly, the embossed line 12b formed on the right side of the longitudinal center line CL is formed such that a virtual line K2 extended from the front end portion to the other side across the left side of the longitudinal center line CL becomes substantially parallel with a tangent line S2 of the rear end portion of an embossed line 10a formed on the left side of the longitudinal center line CL in the embossed portions corresponding to the blood discharge opening 10. Each of angular differences between the virtual lines K1 and K2 and the tangent lines Si and S2 is approximately within 109- or preferably within

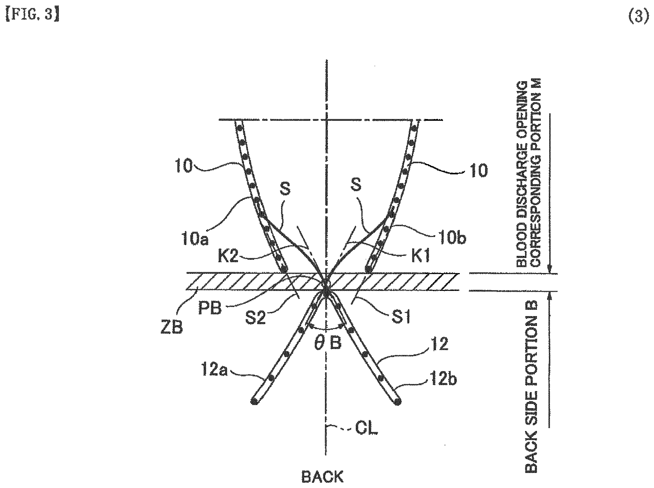

In the back side portion B where the movement of the body is relatively large, in addition to the formation that the separation width WB on the rear end portions is smaller than the separation width WF on the front end portions of the pair of right and left embossed portions corresponding to the blood discharge opening 10 and 10 as described above, the virtual lines K1 and K2 and the tangent lines Si and S2 are formed so that they become substantially parallel, respectively, whereby, as illustrated in Fig. 3, the back side portion B is deformed along the valley of the buttocks. As a result, wrinkles S and S having developed in the direction of the tangent line to the blood discharge opening corresponding portion M side beyond the separation base point PB of the back side embossed portion 12 can be absorbed by the embossed portions corresponding to the blood discharge opening 10. Then, wrinkles are no longer formed in the portions surrounded by the embossed portions corresponding to the blood discharge opening 10 and 10, and the blood discharge opening corresponding portion M can be made to fit the blood discharge opening portion R1 and the perineal region R2 more reliably.

The separation width WF of the front end portions of the embossed portions corresponding to the blood discharge opening 10 and 10 is preferably set to 24 to 35 mm, while the separation width WB of the rear end portions is preferably set to 15 to 20 mm.

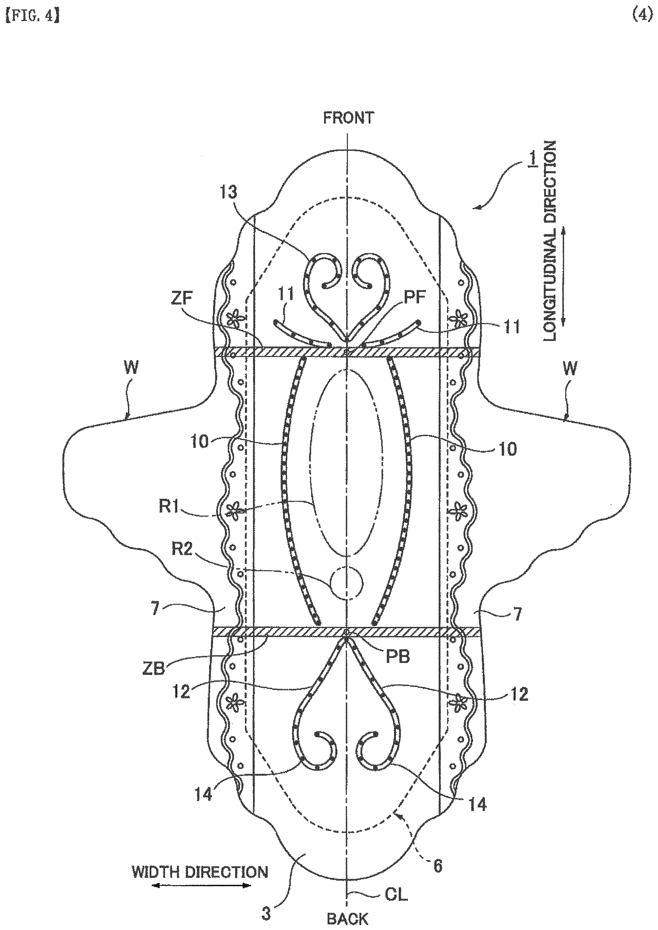

The front side embossed portion 11 is formed by an arc-shaped curve having the center of curvature on the side closer to the front side of the sanitary napkin 1 than the front side embossed portion 11 and formed having an angle OF formed by the tangent lines ha and llb of the both left and right end portions of not less than 90° or preferably 100 to 160°. As a result, the front side portion F can be made easily deformable with the shape along the curve around the pubes of the wearer by using the front side embossed portion 11 as the flexible axis. The front side embossed portion 11 may be formed so as to be divided into plural in the embossed line direction as illustrated in Fig. 4.

The back side embossed portion 12 is formed having an angle θB formed by the embossed lines 12a and 12b formed on the both sides across the longitudinal center line CL of less than 90° or preferably 60 to 90°. As a result, the back side portion B can be made easily deformable with the shape along the valley of the buttocks of the wearer by using the back side embossed portion 12 as the flexible axis. The back side embossed portion 12 may be formed linearly on the both sides toward the back side from the longitudinal center line CL but as in the illustrated example, it is formed having a gentle curved shape with the center of curvature on the outer side of the sanitary napkin 1.

Each of the separation zones ZF and ZB needs to ensure some length in the longitudinal direction with the purpose of separation without having the deformation of the front side portion F and the back side portion B affecting the blood discharge opening corresponding portion M, while if the length is longer than necessary, that prevents fitting to the shape of the body, and the separation zones ZF and ZB become a source of generating wrinkles to the contrary. Thus, the length in the longitudinal direction is set to 1 to 10 mm or preferably to 1 to 5 mm. The separation zones ZF and ZB are preferably located at folding line positions for individually packaging the sanitary napkin 1 by folding twice.

The separation zone ZF (separation base point PF) between the blood discharge opening corresponding portion M and the front side portion F is preferably located closer to the front end portion of the labia pudenda of the blood discharge opening portion R1, while the separation zone ZB (separation base point PB) between the blood discharge opening corresponding portion M and the back side portion B is preferably located closer to the start portion of the valley in the buttocks from the perineal region. As a result, the effect of the fitting performance of each of the parts M, F, and B can be exerted more easily. For this reason, a distance L between the separation zones ZF and ZB (separation base points PF and PB) is set to 60 to 120 mm or preferably to 80 to 90 mm.

As illustrated in Fig. 4, on the front side of the front side embossed portion 11, a design embossed 13 having a shape of a heart expanding to the front side and the front end portion thereof is curved inward or the like can be provided with the purpose of distracting the feeling of gloom during a menstrual period. Moreover, design embossed 14 and 14 continuing from the rear end of the back side embossed portion 12 and curved inward can be made. The design embossed 13 and 14 can conjure an image to a wearer that menstrual blood is stemmed and leakage can be prevented by curving distal end portions inward.

In the illustrated examples, embossed line formation areas are indicated by double lines, and (black dotted) parts indicate high compression portions and the other parts for low compression portions. An interval between the high compression portions (black dots) is arbitrary.

### Reference Signs List

- 1: sanitary napkin
- 2: impermeable back-surface sheet
- 3: permeable front-surface sheet
- 4: absorbing body
- 5: crepe paper
- 6: absorbing element
- 7: side unwoven cloth
- 10: blood discharge opening corresponding portion emboss
- 11: front side embossed portion
- 12: back side embossed portion
- 13, 14: design emboss blood discharge opening corresponding portion front side portion back side portion
ZF, ZB separation zone
PF, PB separation base point

FIG. 1
   (FRONT SIDE)
   LONGITUDINAL DIRECTION
   WIDTH DIRECTION
   (BACK SIDE)
FIG. 2
   FRONT SIDE PORTION F
   COMPRESSION FORCE FROM FEMORAL REGION
   BLOOD DISCHARGE OPENING CORRESPONDING PORTION M
   BACK SIDE PORTION B

## Claims

1. An absorptive article (1) in which an absorbing element is interposed between a permeable front-surface sheet (3) and an impermeable back-surface sheet (2), **characterized in that** a pair of right and left embossed portions (10) corresponding to a blood discharge opening is formed by an arc-shaped curve along a substantially longitudinal direction of the absorptive article and having a center of curvature on the center line side in the longitudinal direction on each of both sides of the longitudinal center line (CL), on a portion corresponding to the blood discharge opening which portion corresponds to a blood discharge opening portion and a perineal region of a wearer when wearing, a front side embossed portion (11) in a substantially width direction of the absorptive article is formed on a front side portion (F) located on the front side with a separation width (DF) provided in the longitudinal direction of the absorptive article from the embossed portion (10) corresponding to the blood discharge opening ,
a discontinuous zone (ZF) of the embossed line where an embossed line is separated in the longitudinal direction between the embossed portion corresponding to the blood discharge opening (10) and the front side embossed portion (11) by the separation width (DF), is formed across a width direction of the absorptive article, and
a back side embossed portion (12) having a substantially inverted V-shape gradually expanding rearward to both sides from a position (PB) on the longitudinal center line (CL), is formed on a back side portion (B) located on the back side with a separation width (DB) provided in the longitudinal direction of the absorptive article from the embossed portion (10) corresponding to the blood discharge opening,
a discontinuous zone (ZB) of the embossed line where an embossed line is separated in the longitudinal direction between the embossed portion (10) corresponding to the blood discharge opening and the back side embossed portion (12) by the separation width (DB), is formed across a width direction of the absorptive article; and
the pair of right and left embossed portions (10) corresponding to the blood discharge opening has front end portions and rear end portions provided separately from each other on both sides of the longitudinal center line(CL), respectively, and the separation width (WB) between the rear end portions is smaller than the separation width (WF) between the front end portions, and the front side embossed portion (11) is formed by an arc-shaped curve having a center of curvature on the side closer to the front side of the absorptive article than the front side embossed portion (11),
an angle formed by tangent lines (11a and 11b) of both right and left end portions is 90° or more and
embossed lines of the back side embossed portion (12) which extend on both sides from the position (PB) on the longitudinal center line (CL) to the back side, have a curved shape having a center of curvature at an outer side of the absorptive article.

2. The absorptive article according to claim 1, wherein in the substantially inverted V-shape embossed portion (12) constituting the back side embossed portion each of the embossed lines (12a and 12b) formed on both sides on the longitudinal center line (CL) is formed such that a virtual line (K1 or K2) extended from the front end portion of the embossed line on one side to the other side across the longitudinal center line (CL) becomes substantially parallel with a tangent line (S1 or S2) of the rear end portion of the embossed line (10a) formed on the other side of the longitudinal center line (CL) of the embossed portion corresponding to the blood discharge opening (10).

3. The absorptive article according to claim 1, wherein in the back side embossed portion (12) an angle formed by the embossed lines (12a and 12b) formed on the both sides across the longitudinal center line (CL), is less than 90°.

## Patentansprüche

1. Absorbierender Artikel (1), bei dem ein absorbierendes Element zwischen eine durchlässige vordere Oberflächenlage (3) und eine undurchlässige hintere Oberflächenlage (2) eingefügt ist, **dadurch gekennzeichnet, dass**
ein Paar von rechten und linken geprägten Bereichen (10), die einer Blutaustrittsöffnung entsprechen, durch eine bogenförmige Linie entlang im Wesentlichen der Längsrichtung des absorbierenden Artikels ausgebildet ist und einen Krümmungsmittelpunkt auf der Mittellinienseite in Längsrichtung auf jeder der beiden Seiten der längs verlaufenden Mittellinie (CL) in einem der Blutaustrittsöffnung entsprechenden Bereich, welcher einem Blutaustrittsöffnungsbereich und einer Perinealregion eines Trägers beim Tragen entspricht, aufweist,
ein geprägter Vorderseitenbereich (11) im Wesentlichen in Richtung der Breite des absorbierenden Artikels in einem auf der vorderen Seite befindlichen Vorderseitenbereich (F) ausgebildet ist, wobei eine Abstandsbreite (DF) in Längsrichtung des absorbierenden Artikels (ausgehend) von dem der Blutaustrittsöffnung entsprechenden geprägten Bereich (10) vorgesehen ist,
eine diskontinuierliche Zone (ZF) der geprägten Linie, dort wo eine geprägte Linie in Längsrichtung zwischen dem der Blutaustrittsöffnung entsprechenden geprägten Bereich (10) und dem geprägten Vorderseitenbereich (11) durch die Abstandsbreite (DF) getrennt ist, über eine Breitenrichtung des absorbierenden Artikels ausgebildet ist, und
ein geprägter Hinterseitenbereich (12), der eine im Wesentlichen umgedrehte V-Form aufweist, die sich von einer Position (PB) auf der längs verlaufenden Mittellinie (CL) nach hinten auf beide Seiten graduell erweitert, in einem auf der hinteren Seite befindlichen Hinterseitenbereich (B) ausgebildet ist, wobei eine Abstandsbreite (DB) in Längsrichtung des absorbierenden Artikels (ausgehend) von dem der Blutaustrittsöffnung entsprechenden geprägten Bereich (10) vorgesehen ist,
eine diskontinuierliche Zone (ZB) der geprägten Linie, dort wo eine geprägte Linie in Längsrichtung zwischen dem der Blutaustrittsöffnung entsprechenden geprägten Bereich (10) und dem geprägten Hinterseitenbereich (12) durch die Abstandsbreite (DB) getrennt ist, über eine Breitenrichtung des absorbierenden Artikels ausgebildet ist, und das Paar von rechten und linken geprägten Bereichen (10), die der Blutaustrittsöffnung entsprechen, vordere Endbereiche und hintere Endbereiche aufweist, die getrennt voneinander auf beiden Seiten der längs verlaufenden Mittellinie (CL) bereitgestellt sind, und die Abstandsbreite (WB) zwischen den hinteren Endbereichen kleiner als die Abstandsbreite (WF) zwischen den vorderen Endbereichen ist, und
der geprägte Vorderseitenbereich (11) durch eine bogenförmige Linie gebildet ist, die einen Krümmungsmittelpunkt auf der Seite aufweist, die näher an der vorderen Seite des absorbierenden Artikels liegt als der geprägte Vorderseitenbereich (11), wobei ein Winkel, der durch die Berührungslinien (11a und 11b) sowohl des rechten als auch des linken Endbereichs gebildet wird, 90° oder mehr beträgt, und
die geprägten Linien des geprägten Hinterseitenbereichs (12), die sich auf beiden Seiten von der Position (PB) auf der längs verlaufenden Mittellinie (CL) zu der hinteren Seite erstrecken, eine gekrümmte Form mit einem Krümmungsmittelpunkt an einer äußeren Seite des absorbierenden Artikels aufweisen.

2. Absorbierender Artikel nach Anspruch 1, wobei in dem eine im Wesentlichen umgedrehte V-Form aufweisenden geprägten Bereich (12), der den geprägten Hinterseitenbereich bildet, jede der auf beiden Seiten der längs verlaufenden Mittellinie (CL) ausgebildeten geprägten Linien (12a und 12b) so ausgebildet ist, dass eine virtuelle Linie (K1 oder K2), die von dem vorderen Endbereich der geprägten Linie auf einer Seite quer über die längs verlaufende Mittellinie (CL) zu der anderen Seite verlängert wird, zu einer Berührungslinie (S1 oder S2) des hinteren Endbereichs der geprägten Linie (10a), die auf der anderen Seite der längs verlaufenden Mittellinie (CL) des der Blutaustrittsöffnung entsprechenden geprägten Bereichs (10) ausgebildet ist, im Wesentlichen parallel wird.

3. Absorbierender Artikel nach Anspruch 1, wobei in dem geprägten Hinterseitenbereich (12) ein von den geprägten Linien (12a und 12b) gebildeter Winkel, der auf den beiden Seiten quer über die längs verlaufende Mittellinie (CL) ausgebildet ist, weniger als 90° beträgt.

## Revendications

1. Article absorbant (1) dans lequel un élément absorbant est intercalé entre une couche de surface supérieure (3) perméable et une couche de surface arrière (2) imperméable, **caractérisé en ce**
**qu'**une paire de parties gaufrées droite et gauche (10) correspondant à une ouverture d'écoulement de sang est formée par une courbe en forme d'arc dans un sens sensiblement longitudinal de l'article absorbant et ayant un centre de courbure du côté de la ligne centrale dans le sens longitudinal sur chacun des deux côtés de la ligne centrale (CL) longitudinale, sur une partie correspondant à une partie d'ouverture d'écoulement de sang, laquelle partie correspond à une partie d'ouverture d'écoulement de sang et une région périnéale d'un utilisateur lorsqu'il le porte,
une partie gaufrée (11) du côté avant dans un sens sensiblement de la largeur de l'article absorbant est formée sur une partie du côté avant (F) située sur le côté avant avec une largeur de séparation (DF) fournie dans le sens longitudinal de l'article absorbant à partir de la partie gaufrée (10) correspondant à l'ouverture d'écoulement de sang,
une zone discontinue (ZF) de la ligne gaufrée dans laquelle une ligne gaufrée est séparée dans le sens longitudinal entre la partie gaufrée (10) correspondant à l'ouverture d'écoulement de sang et la partie gaufrée (11) du côté avant par la largeur de séparation (DF), est formée dans le sens de la largeur de l'article absorbant, et
une partie gaufrée (12) sur l'arrière ayant une forme sensiblement en V inversé se prolongeant progressivement vers l'arrière des deux côtés d'une position (PB) sur la ligne centrale (CL) longitudinale est formée sur une partie du côté arrière (B) située sur le côté arrière avec une largeur de séparation (DB) fournie dans le sens longitudinal de l'article absorbant à partir de la partie gaufrée (10) correspondant à l'ouverture d'écoulement de sang,
une zone discontinue (ZB) de la ligne gaufrée dans laquelle une ligne gaufrée est séparée dans le sens longitudinal entre la partie gaufrée (10) correspondant à l'ouverture d'écoulement de sang et la partie gaufrée (12) sur l'arrière par la largeur de séparation (DB) est formée dans un sens de la largeur de l'article absorbant, et
la paire de parties gaufrées droite et gauche (10) correspondant à l'ouverture d'écoulement de sang a des parties d'extrémité avant et des parties d'extrémité arrière fournies séparément les unes des autres respectivement sur les deux côtés de la ligne centrale (CL) longitudinale, et la largeur de séparation (WB) entre les parties d'extrémité arrière est inférieure à la largeur de séparation (WF) entre les parties d'extrémité avant, et
la partie gaufrée (11) du côté avant est formée par une courbe en forme d'arc ayant un centre de courbure sur le côté le plus proche du côté avant de l'article absorbant que la partie gaufrée (11) du côté avant,
un angle formé par les lignes tangentes (11a et 11b) de chaque partie d'extrémité avant et arrière est supérieur ou égal à 90° et
les lignes gaufrées de la partie gaufrée (12) du côté arrière qui se prolongent sur les deux côtés depuis la position (PB) sur la ligne centrale (CL) longitudinale vers le côté arrière, ont une forme incurvée ayant un centre de courbure au niveau d'un côté extérieur de l'article absorbant.

2. Article absorbant selon la revendication 1, dans lequel dans la partie gaufrée (12) sensiblement en forme de V inversé constituant la partie gaufrée du côté arrière, chaque ligne gaufrée (12a et 12b) formée des deux côtés sur la ligne centrale (CL) longitudinale est formée de telle sorte qu'une ligne virtuelle (K1 ou K2) se prolongeant depuis la partie d'extrémité avant de la ligne gaufrée sur un côté vers l'autre côté à travers la ligne centrale (CL) longitudinale devient sensiblement parallèle à une ligne tangente (S1 ou S2) de la partie d'extrémité arrière de la ligne gaufrée (10a) formée sur l'autre côté de la ligne centrale (CL) longitudinale de la partie gaufrée correspondant à l'ouverture de l'écoulement de sang (10).

3. Article absorbant selon la revendication 1, dans lequel dans la partie gaufrée (12) du côté arrière, un angle formé par les lignes gaufrées (12a et 12b) formées des deux côtés à travers la ligne centrale (CL) longitudinale est inférieur à 90°.
